# EUROPEAN PATENT SPECIFICATION

(11) **EP 3 363 816 B1**
(45) Date of publication and mention of the grant of the patent: **18.11.2026**
(21) Application number: 15906047.4
(22) Date of filing: 15.10.2015
(51) Int. Cl.: C07K 16/28, C12N 15/13, C12N 15/63, A61K 39/395, A61P 35/00, A61K 39/00

(54) **ANTI-OX40 ANTIBODY AND APPLICATION THEREOF**
ANTI-OX40-ANTIKÖRPER UND VERWENDUNGEN DAVON
ANTICORPS ANTI-OX40 ET SON APPLICATION

(43) Date of publication of application: 22.08.2018
(73) Proprietor: Shihuida Pharmaceutical Group (Jilin) Co., Ltd., 134399 Jilin Province (CN)
(72) Inventor: XU, Ting, Suzhou Jiangsu 215125 (CN); LUAN, Yan, Suzhou Jiangsu 215125 (CN); WANG, Xiaoxiao, Suzhou Jiangsu 215125 (CN); PENG, Jianjian, Suzhou Jiangsu 215125 (CN); MA, Shuli, Suzhou Jiangsu 215125 (CN); MA, Hui, Suzhou Jiangsu 215125 (CN); PAN, Xiaolong, Suzhou Jiangsu 215125 (CN); FU, Shilong, Suzhou Jiangsu 215125 (CN); NING, Shanshan, Suzhou Jiangsu 215125 (CN); FEI, Yeqiong, Suzhou Jiangsu 215125 (CN); ZHAO, Meng, Suzhou Jiangsu 215125 (CN)
(74) Representative: Elzaburu S.L.P.
(86) International application number: PCT/CN2015/091958
(87) International publication number: WO 2017/063162

(56) References cited:
- WO-A1-2009/079335
- WO-A1-2014/148895
- WO-A1-2015/153513
- WO-A2-03/106498
- WO-A2-2012/027328
- CN-A- 103 717 263
- CN-A- 104 080 809

## Description

### FIELD OF THE INVENTION

The present invention relates to the field of medical biology, in particular to an anti-OX40 antibody and application thereof. The invention is defined by the appended claims 1-3. Any subject matter in the description not encompassed by the claims is for illustrative purposes and is not part of the invention.

### BACKGROUND OF THE INVENTION

T cell activation requires synergism between two signals: the first signal is generated by recognizing production of an antigen by a T cell antigen receptor (TCR) and is transduced into the cell via a CD3 molecule, the first signal determines the specificity of T cells in adaptive immune response; the second signal is generated by the interaction between co-stimulatory molecules on the surface of antigen-presenting cells (APCs) or target cells with corresponding co-stimulatory molecule receptors on the surface of T cells. Co-stimulatory signals stimulate antigen-specific T cells to proliferate and differentiate into effector T cells. In the absence of co-stimulatory signals, T cells will enter the non-responsive or autoimmune-tolerance state and even enter into programmed death.

OX40, also known as TNFRSF4, ACT35, CD134, IMD16 or TXGP1L, is a member of the TNFR receptor superfamily and is a type I transmembrane glycoprotein. OX40 is mainly expressed on activated CD4 + T cells and CD8 + T cells (Paterson et al. (1987) Mol Immunol 24: 1281-1290). The extracellular segment of OX40 consists of three cysteine-rich domains and one C-terminal incomplete CRD (Deanne M et al. (2006) Structure 14: 1321-1330). OX40 is a secondary co-stimulatory molecule. Unlike CD28, OX40 is not expressed on the surfaces of resting T-cells but is highly expressed 24-72 hours after T-cell activation. The ligand OX40L (TNFSF4, TXGP1, OX-40L, gp34 or CD252) of OX40 is a type II transmembrane glycoprotein and is expressed on activated antigen presenting cells such as dendritic cells, B cells, etc. (Godfrey, WR et al. (1994) J Exp Med 180: 757-762). The OX40/OX40L signal plays a very important role in the activation, proliferation and apoptosis inhibition of T cells. Studies show that activated OX40 antibodies can effectively promote the proliferation and activation of T cells and produce better anti-tumor effects (Brendan D. Curti et al. (2013) Cancer Res 73: 7189-7198).

The use of OX40-activating antibodies in combination with other methods of treating tumors is reviewed and described in detail by Stefanie N. Linch et al. (Stefanie N. Linch et al. (2015) frontiers in oncology 5: 1-14). It has also been reported that the survival of mice can be significantly prolonged when OX40-activating antibodies are used in combination with radiotherapy (Gough M J et al. (2010) J Immunother 33 (8): 798-809; Kjaergaard J et al. (2005) 103 (1): 156-164).

WO 2012/027328 discloses agonist anti-OX40 antibodies that bind to both human and rhesus OX40 and are characterized by specific CDR sequences.

WO 03/106498 discloses fully human agonistic anti-OX40 antibodies based on an OX40-binding scFv fragment with a unique CDR configuration.

WO 2009/079335 provides an isolated binding molecule that competes for binding to human OX40R and is characterized by its complete and unique VH and VL sequences. The binding molecule is specifically implemented as the antibody termed 11D4, which is also shown to bind cynomolgus OX40R with high affinity and to exert inhibition of tumor growth in prostate, colon, and mammary carcinoma models.

WO 2015/153513 relates to OX40 agonist antibodies that bind to human OX40 with sub-nanomolar affinity and whose binding to respective cells can induce depletion of CD4+ effector T cells or regulatory T cells (Treg).

WO 2014/148895 describes anti-OX40 antibodies that exhibit agonist activity through binding to human CD134 (OX40R) on T-effector cells and/or antagonistic activity through binding to human CD134 on T-regulatory cells. The disclosed antibodies are provided as isolated antibodies that bind human CD134 and comprise a light-chain variable region (VL) with a specific amino acid sequence as well as a heavy-chain variable region (VH) with a specific amino acid sequence, wherein the respective sequence may contain up to three amino-acid substitutions in the framework regions.

There remains a need in the art for anti-OX40 antibodies that are capable of binding with high affinity to OX40 and have OX40 agonist activity.

### SUMMARY OF THE INVENTION

In order to solve the above technical problem, the object of the present invention is to provide an anti-OX40 antibody with good specificity, high affinity and stability and application thereof. The present invention provides a solution as defined in the attached claims.

The first aspect of the present invention relates to a anti-OX40 antibody comprising a heavy chain variable region having a sequence of SEQ ID NO: 79, and a light chain variable region having a sequence of SEQ ID NO: 74.

The anti-OX40 antibody set forth in the first aspect of the present invention is a complete antibody.

The anti-OX40 antibody or the antigen-binding site thereof according to any set forth in the first aspect of the present invention contains heavy chain constant regions selected from IgG, IgM, IgE, IgD and IgA.

In an embodiment of the present invention, the heavy chain constant regions are selected from IgGl, IgG2, IgG3 and IgG4.

In an embodiment of the present invention, the heavy chain constant regions are IgGl or IgG4.

The anti-OX40 antibody or the antigen-binding site thereof according to any one set forth in the first aspect of the present invention contains κ or λ light chain constant regions.

Also disclosed, but not part of the scope of the present invention is a nucleic acid molecule, containing a nucleic acid sequence capable of encoding heavy chain variable regions of an antibody, and each heavy chain variable region contains an amino acid sequence selected from the following:
(1) SEQ ID NO: 5-7;
(2) SEQ ID NO: 23-25;
(3) SEQ ID NO: 41-43;
(4) a sequence a) binding to the same epitope as and/or b) being 70%, 80%, 85%, 90% or 97% identical to (1) -(3).

Furthermore, a heavy chain variable region having a sequence of SEQ ID NO: 79 is contained.

In an embodiment of the present invention, the heavy chain constant regions are selected from IgGl, IgG2, IgG3 and IgG4.

In an embodiment of the present invention, the heavy chain constant regions are IgGl or IgG4.

Also disclosed, but not part of the scope of the present invention is a nucleic acid molecule, containing a nucleic acid sequence capable of encoding light chain variable regions of an antibody, and each light chain variable region contains an amino acid sequence selected from the following:
(1) SEQ ID NO: 14-16;
(2) SEQ ID NO: 32-34;
(3) SEQ ID NO: 50-52;
(4) a sequence a) binding to the same epitope as and/or b) being 70%, 80%, 85%, 90% or 97% identical to (1) -(3).

Furthermore, the light chain variable region having a sequence of SEQ ID NO: 74 is contained.

Also disclosed, but not part of the scope of the present invention is a vector containing the nucleic acid molecule as disclosed herein.

Also disclosed, but not part of the scope of the present invention is a host cell, containing the nucleic acid molecule as disclosed herein or the vector as disclosed herein.

Also disclosed, but not part of the scope of the present invention is a conjugate, containing the anti-OX40 antibody or the antigen-binding site thereof as disclosed herein, and other biologically active substances, the anti-OX40 antibody or antigen-binding site thereof is conjugated, either directly or through a linker, to the other biologically active substances.

The other biologically active substances are selected from chemical substances, polypeptides, enzymes, cytokines or other biologically active single substances or mixed substances, such as interleukins, tumor necrosis factors, chemokines, nanoparticles and the like, which can indirectly inhibit the growth of tumor cells or inhibit or kill tumor cells by activating the body's immune response so as to treat tumors.

A further aspect of the present invention relates to a composition (e.g., a pharmaceutical composition), containing the anti-OX40 antibody according to the first aspect of the present invention, and optionally a pharmaceutically acceptable carrier or excipient, and optionally other biologically active substances. The composition according to this aspect of the present invention (e.g., a pharmaceutical composition), the other biologically active substances include but are not limited to other antibodies, fusion proteins or drugs (e.g., anti-tumor drugs such as radiotherapy or chemotherapy drugs).

The present invention also relates to the use of the anti-OX40 antibody according to any set forth in the first aspect of the present invention, or the composition according to the above aspect for the preparation of a medicament for the prevention or treatment of tumors. Any references in the description to methods of treatment refer to the compounds, pharmaceutical compositions and medicaments of the present invention for use in a method for treatment of the human (or animal) body by therapy (or for diagnosis).

With the above-described solutions, the present invention has at least the following advantages:

In the present invention, an anti-OX40 antibody is obtained by means of screening with a yeast display technology and further affinity maturation, the anti-OX40 antibody has good specificity, high affinity and stability, can specifically bind to human OX40, and can enhance the activation of T cells through binding to activated T cell and thus has a significant inhibitory effect on tumor growth. In addition, the anti-OX40 antibody of the present invention is a fully human antibody, which has a lower immunogenicity, less rejection in patients, and better drug-forming potential than the conventional murine, chimeric and humanized antibodies.

The above description is merely a summary of the technical solutions of the present invention. In order to understand the technical solutions of the present invention more clearly and implement the solutions according to the description, further examples illustrating the present invention will be described in detail below with reference to the accompanying drawings.

### BRIEF DESCRIPTION OF THE DRAWINGS

Figure 1 is a graph showing the results of the binding of purified anti-hOX40 scFv to hOX40,
   where the x-axis indicates the fluorescence intensity of EGFP and the y-axis indicates the fluorescence intensity of anti-hlg-PE;
Figure 2 shows antibody specificity detection of the anti-OX40,
   where the x-axis indicates the fluorescence intensity of EGFP and the y-axis indicates the fluorescence intensity of anti-hlg-PE;
Figure 3 is a graph showing the staining results of yeast monoclones expressing antibodies with improved affinity;
Figure 4 is a graph showing the binding capacity of three antibodies to hOX40 using ELISA detection;
Figure 5 is a graph showing the binding capacity of different heavy chain mutants to hOX40 after affinity maturation using ELISA detection;
Figure 6 is a graph showing the binding capacity of H96 heavy chain mutant and different light chain mutants to hOX40 after affinity maturation using ELISA detection;
Figure 7 is a graph showing the results of the *in vitro* agonist activity detection of anti-hOX40 antibodies;
Figure 8 is a graph comparing the *in vitro* agonist activity of three IgG subtypes of H96-L80;
Figure 9 is a graph showing the binding capacity of anti-OX40 antibodies to CD4+ T and CD8+ T cells in humans and macaques;
Figure 10 is a graph showing the results of activity detection of O21 scFv antibody agonist;
Figure 11 shows the results of activity detection of O21 scFv and IgG agonists;
Figure 12 shows the *in vitro* effect of IgGl and IgG4 subtypes of H96-L80 on T cell subtypes;
Figure 13 is a graph showing the *in vivo* growth inhibition effect of O21 mAb on a tumor (PC-3);
Figure 14 is a graph showing the *in vivo* growth inhibition effect of O21 mAb on a tumor (A375);
Figure 15 is a graph showing the stability of anti-OX40 antibodies evaluated by a 45 °C accelerated stability test,
Where A is concentration measurement, B is monomer content measurement, C is *in vitro* activity detection using an NF-κb system;
Figure 16 shows the pharmacokinetic results of the H96-L80 antibody.

### DETAILED DESCRIPTION OF THE EMBODIMENTS

Specific embodiments of the present invention are described in further details below with reference to the figures and embodiments. The following embodiments illustrate the present invention but are not intended to limit the scope of the present invention.

The present invention is further described as below: in the present invention, unless otherwise specified, the scientific and technical terms used herein have the meanings commonly understood by those skilled in the art. Also, the protein and nucleic acid chemistry, molecular biology, cell and tissue culture, microbiology, immunology-related terms, and laboratory procedures used herein are terms and routine steps that are widely used in the relevant art. In the meantime, in order to better understand the present invention, definitions and explanations of related terms are provided below.

In the present invention, the term "antibody" refers to an immunoglobulin molecule usually composed of two identical pairs of polypeptide chains each having one "light" (L) chain and one "heavy" (H) chain. The light chains of an antibody can be classified as κ and λ light chains. The heavy chains can be classified as µ, δ, γ, α or ε, and the isotypes of an antibody are defined as IgM, IgD, IgG, IgA and IgE, respectively. Within the light and heavy chains, the variable and constant regions are joined by a "J" region of about 12 or more amino acids, and the heavy chains further comprise a "D" region of about 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain, CL. The constant region of an antibody may mediate binding of the immunoglobulin to the host tissue or factor, including various cells of the immune system (e.g., effector cells) and the first component (Clq) of the classical complement system. The VH and VL regions can also be subdivided into regions with high variability known as complementarity determining regions (CDRs) interspersed with more conserved regions known as framework regions (FRs). Each VH and VL consists of 3 CDRs and 4 FRs arranged from N-terminal to C-terminal in the following order: FR1, CDR1, FR2, CDR2, FR3, CDR3, FR4. The variable regions (VH and VL) of each heavy/light chain pair form the antibody binding site, respectively. Distribution of amino acids to regions or domains follows the definition of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883. The amino acid positions described in the present invention are based on an online comparison of abysis tools (http://www.bioinf.org.uk/abysis/index.html) and do not represent actual positions in the amino acid sequence.

The term "antibody" is not limited by any particular antibody-producing method. For example, it includes, in particular, recombinant antibodies, monoclonal antibodies and polyclonal antibodies. Antibodies may be antibodies of different isotypes, e.g., IgG (e.g., IgGl, IgG2, IgG3 or IgG4 subtype), IgAl, IgA2, IgD, IgE or IgM antibody.

In the present invention, the term "antigen-binding site" of an antibody refers to one or more sites of a full-length antibody that retains the ability to bind the same antigen to which the antibody binds (e.g., OX40) and competes against an intact antibody for an antigen specific binding. See generally, Fundamental Immunology, Ch. 7 (Paul, W., ed., 2nd., Raven Press, N.Y. (1989). Antigen-binding sites can be produced by recombinant DNA techniques or by enzymatic or chemical cleavage of intact antibodies. In some cases, the antigen binding site includes Fab, Fab ', F (ab') 2, Fd, Fv, dAb and complementarity determining region (CDR) fragments, single chain antibodies (e.g., scFv), chimeric antibodies, diabodies, and polypeptides that comprise at least a portion of an antibody that is sufficient to confer specific antigen-binding ability to the polypeptide.

In the present invention, the term "vector" refers to a nucleic acid vehicle into which a polynucleotide encoding a protein can be inserted and expressed. The genetic material elements carried in the vector can be expressed in a host cell by transforming, transducing, or transfecting the host cell with the vector. Embodiments of vectors include: plasmids; phagemids; cosmos; artificial chromosomes such as yeast artificial chromosomes (YACs), bacterial artificial chromosomes (BACs) or P1-derived artificial chromosomes (PACs); phages such as λ phage or M13 phage and animal viruses. Animal virus used as a carrier include retrovirus (including lentivirus), adenovirus, adeno-associated virus, herpes virus (such as herpes simplex virus), jaundice virus, baculovirus, papilloma virus, papovaviruses (such as SV40). A vector may contain a variety of elements that control expression, including promoter sequences, transcriptional initiation sequences, enhancer sequences, selection elements, and reporter genes. In addition, the vector may also contain an origin of replication. It is also possible that the vector may include components that assist its entry into the cell, such as viral particles, liposomes or protein shells, but not only these substances.

In the present invention, the term "host cell" refers to a cell into which a vector is introduced and includes many cell types such as prokaryotic cells such as *Escherichia coli* and *Bacillus subtilis,* fungal cells such as yeast cells or *Aspergillus* cells, insect cells such as S2 Drosophila cells or Sf9, or animal cells such as fibroblasts, CHO cells, COS cells, NSO cells, HeLa cells, BHK cells, HEK293 cells or human cells.

In the present invention, "specifically binding" refers to a non-random binding reaction between two molecules, for example, a reaction between an antibody and an antigen that produces the antibody. Here, the binding affinity of the antibody that binds the first antigen to the second antigen is undetectable or weak. In certain embodiments, an antigen-specific antibody refers to an antibody that binds to the antigen with an affinity (KD) ≤10⁻⁵ M (e.g. 10⁻⁶ M, 10⁻⁷ M, 10⁻⁸ M, 10⁻⁹ M, 10⁻¹⁰ M, etc.), where KD refers to the ratio of dissociation rate to binding rate (k_{off}/kₒₙ), which can be determined using methods familiar to one skilled in the art.

### Embodiment 1: Expression of Recombinant Human OX40 and Preparation of Related EGFP Cells

S1.1 The amino acid sequence of the human OX40 (hOX40) extracellular domain (i.e., the residue 1 to the residue 216 in P43489) was obtained based on the amino acid sequence of human OX40 (P43489) in the Uniprot protein database;
S1.2 The amino acid sequence of the human IGG1-Fc (hFc) domain (i.e., the residue 104 to the residue 330 in P01857) was obtained based on the constant region amino acid sequence of human immunoglobulin gamma1 (IgG1) (P01857) in the Uniprot protein database;
S1.3 The amino acid sequence of the mouse IgG1-Fc (muFc) domain (i.e., the residue 98 to the residue 324 in P01868) was obtained based on the constant region amino acid sequence (P01868) of mouse immunoglobulin gamma1 (IgG1) in the Uniprot protein database;
S1.4 The DNA fragments encoding the amino acid sequences in steps S1.1 to S1.3 were obtained by artificial synthesis. The synthesized gene sequences were sub-cloned into the commercial vector pcDNA4/myc-HisA (Invitrogen, V863-20) by double digestion by HindIII and EcoRI (Fermentas), respectively, and the accuracy of the constructed plasmid was verified by sequencing to obtain recombinant the plasmid DNA i.e., pcDNA4-hOX40-hFc and pcDNA4-hOX40-muFc;
S1.5 The amino acid sequences of EGFP (C5MKY7), human OX40 (P43489), mouse OX40 (P47741), human CD137 (Q07011) and human CD27 (P26842) were obtained based on the information in Uniprot protein database;
S1.6 The DNA fragments encoding the amino acid sequences in steps S1.5 were obtained synthetically, the synthesized gene sequences were sub-cloned into the commercial vector pcDNA4/myc-HisA (Invitrogen, V863-20) by double digestion by HindIII and EcoRI (Fermentas), respectively, and the accuracy of the constructed plasmid was verified by sequencing to obtain the recombinant plasmid DNA i.e., pcDNA4-hOX40-EGFP, pcDNA4-hCD137-EGFP, pcDNA4-mOX40-EGFP and pcDNA4-hCD27-EGFP;
S1.7 The EGFP recombinant plasmid in step S1.6 was transfected into HEK293 (ATCC, CRL-1573TM) cells, and the expression of hOX40, hCD137, mOX40 and hCD27 was confirmed by fluorescence activated signal sorting (FACS) 48h after transfection.
S1.8 pcDNA4-hOX40-Fc and pcDNA4-hOX40-muFc were transiently transfected into HEK293 cells for protein production. The recombinant expression plasmids were diluted with FreeStyle293 medium and PEI (polyethylenimine) solution for transformation was added; each group of plasmid/PEI mixture was added to the cell suspension respectively and incubated at 37 °C, 10% CO2 and 90 rpm; after 5-6 days, culture supernatant was collected and purified by Protein A affinity chromatography to obtain hOX40-Fc, hOX40-muFc protein samples for the following embodiments. The obtained protein samples were subjected to preliminary detection by SDS-PAGE, and the target band can be clearly seen.

### Embodiment 2: Screening, cloning, expressing and identification of Anti-hOX40 Antibody from Yeast Display Library

### 2.1 Methods

Yeast display technology was used to screen for the whole human antibodies against human OX40. The scFV yeast display library was constructed by cloning the VH and VL genes in the IgM and IgG cDNA from PBMCs from 150 healthy human beings (The linker sequence between the VH and VL is GGGGSGGGGSGGGGS (SEQ ID NO: 1) linker peptide). The library volume is 5 × 10⁸. The 10-fold volume of yeast library was resuscitated to induce the expression of the antibody on yeast surface; the yeasts were enriched twice with 100 nM antigens for biotinylated hOX40 using magnetic bead sorting, and then further enriched twice using FACS for biotinylated hOX40. The enriched yeasts were coated on the plate, and monoclones were picked from it. After amplification and induction of expression of the monoclonal yeasts, the monoclonal yeasts were analyzed by staining using anti-myc antibody and biotinylated hOX40 or control antigen hCD137. The yeasts with positive antigen/negative control yeasts were identified as positive yeasts.

The yeast clones confirmed by FACS were subjected to yeast colony PCR and sequencing. The PCR primers were: sequence-F: CGTAGAATCGAGACCGAGGAGA (SEQ ID NO: 2); sequence-R: CTGGTGGTGGTGGTTCTGCTAGC (SEQ ID NO: 3). Sequencing primers were sequence-R. After sequencing, BioEdit software was used for sequence alignment and analysis.

The gene of the single-chain antibody scFv obtained above was fused with the above human IgG1-Fc gene, and then double-digested with HindIII and EcoRI (Fermentas) and cloned into the commercial vector pcDNA4/myc-HisA. The cloning and extraction in small amount of the plasmid were carried out according to the standard operation of Molecular Cloning. The extracted plasmid was transiently expressed in HEK293 cells and purified by protein A column.

The hOX40-EGFP cells were resuspended in 0.5% PBS-BSA Buffer, and 2 µg of the above purified anti-hOX40 scFv antibody was added, and the relevant control was set at the same time. The negative control was 2 µg of hIgG1 protein. The secondary antibody is anti-hIg-PE (eBioscience). After staining, flow cytometry was performed. In this way, antibodies that bind to the hOX40 antigen on cell surface were identified.

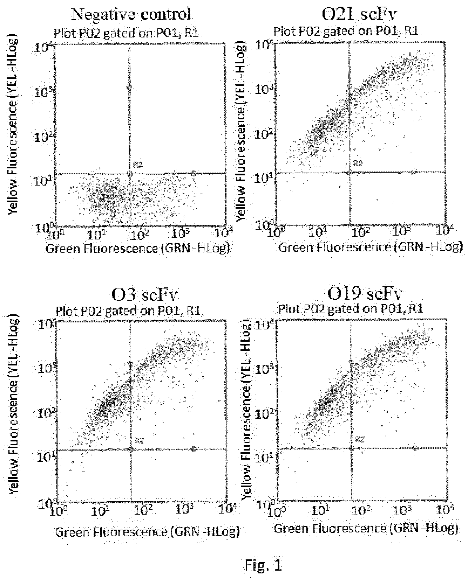

After screening and identification, three better antibodies O3scFv, O19scFv and O21scFv were obtained. As shown in Figure 1, the three anti-hOX40 antibodies were able to bind to the hOX40 on cell surface whereas the negative control was unable to bind to the hOX40 on cell surface. A linker with a sequence of GGGGSGGGGSGGGGS (SEQ ID NO: 1) was between the heavy and light chain variable regions of the above antibodies.

### 2.2 Sequences

2.2.1 The amino acid sequence of each heavy chain variable region of O3 scFv is: 
QVQLQQWGAGLLKPSETLSLTCGFNGEYFTDYFWTWVRQPPGEALEWLALIYWDDDERY
SPSLKNRLIITKDISKNQVVLTMTHMEPADTGTYYCARWGGSLMNAFDVWGPGTMVTVS
S (SEQ ID NO: 4)
 where the underlined parts are CDR1, CDR2 and CDR3, respectively, and the sequence numbers thereof are SEQ ID NOs: 5-7, respectively; the parts without underline are FR1, FR2, FR3 and FR4, respectively, with the sequence numbers of SEQ ID NOs: 8-11, respectively.

The DNA sequence corresponding to the amino acid sequence of each heavy chain variable region of O3 scFv is:

CAGGTGCAGCTACAGCAGTGGGGCGCAGGACTGTTGAAGCCTTCGGAGACCCTGTCCCT
CACCTGCGGTTTCAATGGAGAATACTTCACTGATTACTTCTGGACCTGGGTCCGGCAGC
CCCCCGGAGAGGCCCTGGAGTGGCTTGCACTCATTTATTGGGATGATGATGAGCGCTAC
AGCCCATCTCTGAAGAACAGACTCATCATCACCAAGGACATTTCCAAAAACCAGGTGGT
CCTTACAATGACCCACATGGAGCCTGCGGACACAGGCACCTATTACTGTGCGAGATGGG
GTGGTTCTTTAATGAACGCTTTTGATGTCTGGGGCCCAGGGACAATGGTCACCGTCTCTT
CA (SEQ ID NO: 12)

The amino acid sequence of each light chain variable region of O3 scFv is: 
QSALIQPASVSGSPGQSITISCTGTSSDVGGYNYVSWYQRHPGKAPRLMIYDVTKRPSGVS
NRFSGSKSGNTASLTISGLQAEDEADYYCSSYTSSSIAVFGGGTQLTVL (SEQ ID NO: 13)
 where the underlined parts are CDR1, CDR2 and CDR3, respectively, with the sequence numbers of SEQ ID NOs: 14-16, respectively; the parts without underline are FR1, FR2, FR3 and FR4, respectively, with the sequence numbers of SEQ ID NOs: 17-20, respectively.

the DNA sequence corresponding to the amino acid sequence of each light chain variable region of O3 scFv is:

CAGTCTGCCCTGATTCAGCCTGCCTCCGTGTCTGGGTCTCCTGGACAGTCGATCACCATC

TCCTGCACTGGAACCAGTAGTGACGTTGGTGGTTATAATTATGTCTCCTGGTACCAACG
ACACCCAGGCAAAGCCCCCAGACTCATGATTTATGATGTCACTAAGCGGCCCTCAGGGG
TTTCTAATCGCTTCTCTGGCTCCAAGTCTGGCAACACGGCCTCCCTGACCATCTCTGGGC
TCCAGGCTGAGGACGAGGCTGATTATTACTGCAGCTCATATACAAGCAGCAGCATTGCT
GTGTTCGGAGGAGGCACCCAGCTGACCGTCCTC (SEQ ID NO: 21)

2.2.2 The amino acid sequence of each heavy chain variable region of 019 scFv: 
QVQLVESEGGLVQPGGSLRLSCAASRFTFSNYWMSWVRQAPGKGLEWVANIKQDGSEKY
YMDSVKGRFTISRDNAKNSLFLQMNTLRAEDTAMYYCTRVSFGVPTYDDFWRSYATPA
WYFDFWGRGTLVTVSS (SEQ ID NO: 22)
 where the underlined parts are CDR1, CDR2 and CDR3, respectively, with the sequence numbers of SEQ ID NOs: 23-25, respectively; the parts without underline are FR1, FR2, FR3 and FR4, respectively, with the sequence numbers of SEQ ID NOs:26-29, respectively.

The DNA sequence corresponding to the amino acid sequence of each heavy chain variable region of 019 scFv is:

CAGGTGCAGCTGGTGGAGTCTGAGGGAGGCTTGGTCCAGCCTGGGGGGTCCCTGAGAC
TCTCCTGCGCAGCCTCTAGATTCACGTTTAGTAACTATTGGATGAGCTGGGTCCGCCAG
GCTCCAGGGAAAGGGCTGGAGTGGGTGGCCAATATAAAGCAAGATGGAAGTGAGAAAT
ATTATATGGACTCTGTGAAGGGCCGATTCACCATCTCCAGAGACAACGCCAAGAACTCA
CTGTTTCTGCAGATGAACACCCTAAGAGCCGAGGACACGGCTATGTATTACTGTACGAG

GGTTAGTTTCGGAGTGCCGACGTATGACGATTTTTGGAGGAGTTACGCGACGCCCGCTT
GGTACTTCGATTTTTGGGGCCGTGGTACCCTGGTCACTGTCTCCTCA (SEQ ID NO: 30)

The amino acid sequence of each light chain variable region of 019 scFv is: 
QSALIQPASVSGSPGQSITISCTGISSDDGYYKYVSWYQQYPGKAPKLMIYDVSKRPSGISFR

FSGSKSGNTASLTISGLQAEDEADYYCSSYTSNMTPYVFGTGTKVTVL (SEQ ID NO: 31)
 where the underlined parts are CDR1, CDR2 and CDR3, respectively, with the sequence numbers of SEQ ID NOs: 32-34, respectively; the parts without underline are FR1, FR2, FR3 and FR4, respectively, with the sequence numbers of SEQ ID NOs:35-38, respectively.

The DNA sequence corresponding to the amino acid sequence of each light chain variable region of 019 scFv is:

CAGTCTGCTCTGATTCAGCCTGCCTCCGTGTCTGGGTCTCCTGGACAGTCGATCACCATC
TCCTGCACTGGAATTAGTAGTGACGATGGTTATTATAAGTATGTCTCCTGGTACCAACA
ATATCCAGGCAAAGCCCCCAAACTCATGATTTATGATGTCAGTAAGCGGCCCTCAGGGA
TTTCTTTTCGCTTCTCTGGCTCCAAGTCTGGCAACACGGCCTCCCTGACCATCTCTGGGC
TCCAGGCTGAGGACGAGGCTGATTATTACTGCAGCTCATATACAAGTAACATGACCCCC
TATGTCTTCGGCACTGGGACCAAGGTCACCGTCCTA (SEQ ID NO: 39)

2.2.3 The amino acid sequence of each heavy chain variable region of O21 scFv is: 
QVQLQQSGPGLVKPSQTLSLTCAISGDSVSSNSVSWDWIRQSPSRGLEWLGRTYYRSKWY
NEYAVSVESRITINPDTSKNQFSLQLNSVTPEDTAIYFCVRNNYFFDLWGRGTLVTVSS
(SEQ ID NO: 40)
 where the underlined parts are CDR1, CDR2 and CDR3, respectively, with the sequence numbers of SEQ ID NOs: 41-43, respectively; the parts without underline are FR1, FR2, FR3 and FR4, respectively, with the sequence numbers of SEQ ID NOs:44-47, respectively.

The DNA sequence corresponding to the amino acid sequence of each heavy chain variable region of O21 scFv is:

CAGGTACAGCTGCAGCAGTCAGGTCCAGGACTGGTGAAGCCCTCGCAGACCCTCTCACT
CACCTGTGCCATCTCCGGGGACAGTGTCTCTAGCAACAGTGTCTCTTGGGACTGGATCA
GGCAGTCCCCCTCGAGGGGCCTTGAGTGGCTGGGAAGGACATACTATAGGTCCAAGTG
GTATAATGAGTATGCAGTATCTGTGGAAAGTCGAATAACCATCAACCCAGACACATCCA
AGAACCAGTTCTCCCTGCAACTGAACTCTGTGACTCCCGAGGACACGGCTATATATTTC
TGTGTAAGAAATAACTACTTCTTCGATCTCTGGGGCCGTGGTACCCTGGTCACCGTCTCC
TCA (SEQ ID NO: 48)

The amino acid sequence of each light chain variable region of O21 scFv is: 
EIVLTQSPATLSLSPGERATLSCRASQSVSSYLAWYQQKPGQAPRLLIYDASDRATGIPARFS
GSGSGTDFTLTISSLEPEDFAVYYCQLRSNWPPGYTFGQGTKVEIK（SEQ ID NO：49）
 where the underlined parts are CDR1, CDR2 and CDR3, respectively, with the sequence numbers of SEQ ID NOs: 50-52, respectively; the parts without underline are FR1, FR2, FR3 and FR4, respectively, with the sequence numbers of SEQ ID NOs:53-56, respectively.

The DNA sequence corresponding to the amino acid sequence of each light chain variable region of O21 scFv is:

GAAATTGTGTTGACACAGTCTCCAGCCACCCTGTCTTTGTCTCCAGGGGAAAGAGCCAC

CCTCTCCTGCAGGGCCAGTCAGAGTGTTAGCAGCTACTTAGCCTGGTACCAACAGAAAC

CTGGCCAGGCTCCCAGGCTCCTCATCTATGATGCATCCGACAGGGCCACTGGCATCCCA

GCCAGGTTCAGTGGCAGTGGGTCTGGGACAGACTTCACTCTCACCATCAGCAGCCTTGA

GCCTGAAGATTTTGCAGTTTATTACTGTCAGCTGCGTAGCAACTGGCCTCCGGGGTACA

CTTTTGGCCAGGGGACCAAGGTGGAGATCAAA (SEQ ID NO：57)

### Embodiment 3: scFv antibodies of the three antibodies were formatted as IgG-type antibodies

The amino acid sequence of human IgG4 constant region was obtained based on the amino acid sequence (P01861) of the constant region of human immunoglobulin gamma4 (IgG4) in Uniprot protein database. The gene sequences of the heavy chain variable region of the VH sequences of the screened O3, 019, O21 and the gene sequences of the human IgG4 constant region were spliced together, respectively, and the spliced genes were synthesized and double-digested with HindIII and EcoRI (Fermentas) and subcloned into the vector pcDNA4/myc-HisA to obtain pcDNA4-O21HC, pcDNA4-O3HC, pcDNA4-O19HC.

The amino acid sequence of human κ light constant region was obtained based on the amino acid sequence (P01834) of the κ constant region of human immunoglobulin in Uniprot protein database. The gene sequence of the light chain variable region of the VL sequences of the screened 021 and the gene sequence of human κ light chain constant region were spliced together; The amino acid sequence of human λ light constant region was obtained based on the amino acid sequence (A0M8Q6) of the λ constant region of human immunoglobulin in Uniprot protein database; The gene sequences of the heavy chain variable region of the VL sequences of the screened O3, 019 and the gene sequence of human λ light chain constant region were spliced together, respectively, and the spliced genes were synthesized and double-digested with HindIII and EcoRI (Fermentas) and subcloned into the vector pcDNA4/myc-HisA to obtain pcDNA4-O21LC, pcDNA4-O3LC, pcDNA4-O19LC.

Plasmids comprising the heavy or light chain obtained above were extracted using the plasmid maxi preparation kit (PL14) supplied by AidLab. Recombinant plasmids comprising the heavy or light chain were co-transfected into HEK293 cells for antibody expression. The recombinant expression plasmids were diluted with FreeStyle293 medium and PEI (polyethylenimine) solution for transformation; each group of plasmid/PEI mixture was added to the cell suspension respectively and incubated at 37 °C, 10% CO2 and 120 rpm; after 5-6 days, the transient expression culture supernatant was collected and purified by Protein A affinity chromatography to obtain anti-hOX40 antibodies: O3mAb, O19mAb and O21mAb.

### Embodiment 4: Identification of anti-hOX40Abs

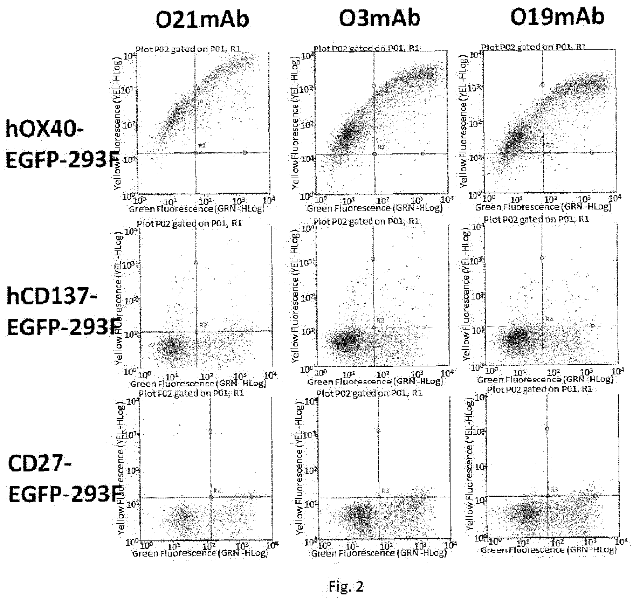

Identification of whether the antibodies specifically recognize hOX40:
Binding of purified anti-hOX40 antibodies to hOX40, hCD137 and hCD27 proteins
HEK293 cells expressing hOX40-EGFP, hCD137-EGFP and hCD27-EGFP obtained in Embodiment 1 were resuspended in 0.5% PBS-BSA Buffer and anti-hOX40 mAb protein was added and the mixture was incubated on ice for 20 min. After washing, secondary antibody anti-hIg-PE (eBioscience) was added, and the mixture was incubated on ice for 20 min. After washing, the cells were resuspended in 500µl 0.5% PBS-BSA Buffer and detected by flow cytometry. As shown in Figure 2, all the three antibodies (O3mAb, O19mAb and O21mAb) can bind to hOX40-EGFP cells but not to several other EGFP cells (hCD137-RGFP-293F, hCD27-RGFP-293F) and very good specificity was shown.

### Embodiment 5: Increased in vitro affinity of anti-OX40 antibody

Since all the three antibodies (O3mAb, O19mAb and O21mAb) were able to specifically bind to hOX40-EGFP cells and O21 scFv was found to have a higher affinity to hOX40 with a stable higher expression level by transient transfection, therefore O21 scFv antibody was selected for further *in vitro* affinity test. However, it should be noted that the purpose of the *in vitro* affinity test is to study the influence of the mutation rate on the antibody affinity. The test result should not be limited to the identification of O21 scFv but should be understood as the influence of the mutation rate on the affinity of antibodies including O3 scFv, 019 scFv and O21 scFv.

### 5.1 Construction of yeast library of anti-OX4021 # ScFv with improved affinity

The standard PCR reaction was performed using the pcDNA4-OX40-21-Fc plasmid constructed in Embodiment 1 as a template, pcDNA4-F:TCTGGTGGTGGTGGTTCTGCTAGC (SEQ ID NO: 58) and cMyc-BBXhoI: GCCAGATCTCGAGCTATTACAAGTCTTCTTCAGAAATAAGCTTTTGTTCTAGAATTCCG (SEQ ID NO: 59) as primers. The resulting PCR products were digested with NheI and BglII from Fermentas to construct a recombinant plasmid. Next, a random mutation PCR product of scFv was obtained by error prone PCR using the method of Ginger et al. (2006) Nat Protoc 1 (2): 755-68. The primers used were ep-F: TAATACGACTCACTATAGGG (SEQ ID NO : 60) and ep-R: GGCAGCCCCATAAACACACAGTAT (SEQ ID N0: 61). The resulting PCR products were purified by the GeneJET DNA purification Kit from Fermentas and then precipitated in ethanol to a concentration greater than 1 µg/µl. The remainder of the procedure references the method of Ginger et al. (2006) Nat Protoc 1 (2): 755-68 to obtain an affinity-matured yeast library using yeast electrotransformation and *in vivo* recombination.

### 5.2 Screening of yeasts expressing anti-OX40 21 # scFv with improved affinity

The affinity-matured yeast library obtained above was subjected to two rounds of FACS with 10 nM and 1 nM hOX40-Fc protein, and the resulting yeast products were plated and subjected to monoclonal identification. The yeast monoclones expressing antibodies with improved affinity were identified by flow cytometry using the method of staining with low concentration of antigen and the wild-type yeast obtained previously as a control. The yeast colonies identified by FACS were subjected to yeast colony PCR and sequencing as above. The obtained sequences were sequenced and analyzed using BioEdit software for mutation sites.

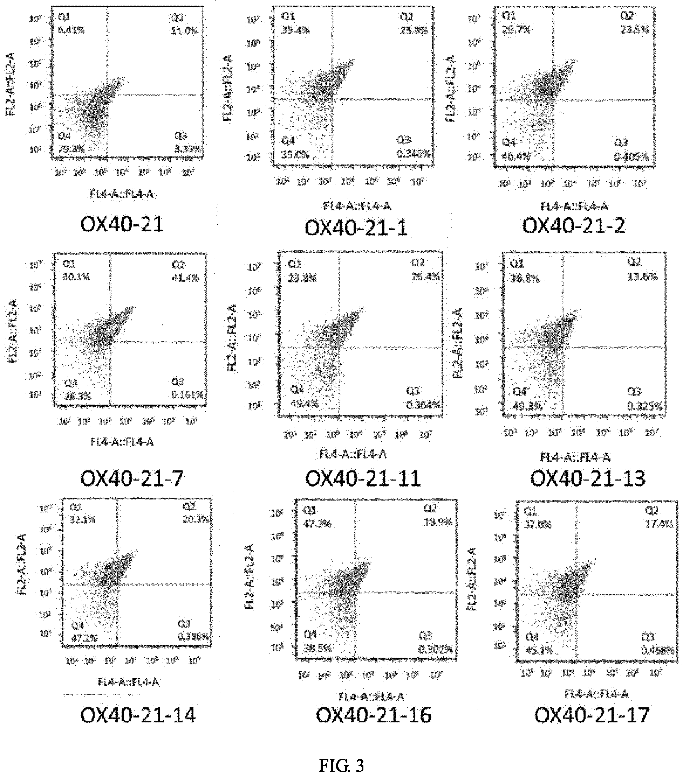

The mutation sites in O21 scFv that did not affect or even improve antibody affinity are shown in Table 1. The results in this table shows that one or more of the mutations in O21 scFv set forth in the table do not affect or even improve affinity; the results of corresponding sequence analysis for selected yeast monoclones are shown in Table 2. This table shows that the mutation sites listed in Table 1, when combined as shown in Table 2, can improve the affinity of the antibody. The staining results of the yeasts shown in Table 2 are shown in Figure 3, and compared with O21 scFv, the affinity of the antibody has been improved to varying degrees.

**Table 1 Mutation sites in O21 scFv with no effect on antibody affinity**

| Heavy chain | Light chain |
|---|---|
| Position H7: S mutated to L | Position L30: S mutated to G |
| Position H13: K mutated to M | Position L90: L mutated to Q |
| Position H28: S mutated to N | |
| Position H33: S mutated to G | |
| Position H43: R mutated to G | |
| Position H65: S mutated to N | |
| Position H67: I mutated to M | |
| Position H96: N mutated to D | |

**Table 2 The results of sequence analysis for yeast monoclones expressing antibodies with improved affinity;**

| Name | Mutation site (heavy chain) | Mutation site (light chain) |
|---|---|---|
| anti-OX40-21-1 | | Position L90: L mutated to Q |

| | | |
|---|---|---|
| anti-OX40-21-2 | Position H43: R mutated to G | |
| | Position H96: N mutated to D | |
| anti-OX40-21-7 | | Position L30: S mutated to G |
| | | Position L90: L mutated to Q |
| anti-OX40-21-11 | Position H7: S mutated to L | Position L30: S mutated to G |
| | Position H96: N mutated to D | |
| anti-OX40-21-13 | Position H28: S mutated to N | |
| | Position H33: S mutated to G | |
| | Position H96: N mutated to D | |
| anti-OX40-21-14 | Position H96: N mutated to D | Position L30: S mutated to G |
| anti-OX40-21-16 | Position H67: I mutated to M | Position L90: L mutated to Q |
| anti-OX40-21-17 | Position H13: K mutated to M | |
| | Position H65: S mutated to N | |
| | Position H96: N mutated to D | |

From Table 2, it has been experimentally shown that the affinity of the antibody can still be maintained or even improved if 3 and 2 amino acids in the heavy chain CDRs and the light chain CDRs of O21 scFv are mutated, respectively (up to 3 amino acids can be mutated in the anti-OX40 21 # H96-L80 mAb in Embodiment 6). It is now known that the heavy chain variable regions and the light chain variable regions of O21 scFv comprise 119 amino acids (wherein the CDRs contain 32 amino acids, the FRs contain 87 amino acids) and 109 amino acids (wherein the CDRs contain 29 amino acids and the FRs contain 80 amino acids), then it can be considered that an antibody with more than 90% homology with the heavy chain CDRs or the light chain CDRs still have the ability to maintain or even improve the affinity of the antibody. In practice, the affinity of an antibody has no decisive relationship with the percentage of sequence homology, but rather affected by the key amino acid residues that constitute the epitope (or antigenic determinant). The sequence homology can be as low as 70% if these key amino acid residues have not been mutated or the mutations thereof do not have played a decisive role in the affinity change.

### Embodiment 6: scFv antibodies were formatted as IgG-type antibodies

Affinity-matured scFv antibodies were formatted as IgG type antibodies according to the method in Embodiment 3 to obtain a series of anti-OX40 21 # mAb variants with the following sequence:

### 6.1 anti-OX40 21#H28H33 mAb

The amino acid sequence of the heavy variable region of anti-OX40 21#H28H33 mAb is: 
QVQLQQSGPGLVKPSQTLSLTCAISGDNVSSNGVSWDWIRQSPSRGLEWLGRTYYRSKWY

NEYAVSVESRITINPDTSKNQFSLQLNSVTPEDTAIYFCVRNNYFFDLWGRGTLVTVSS (SEQ

ID NO: 62)
 where the underlined parts are CDR1, CDR2 and CDR3, respectively, (the parts in the box are the mutated parts), with the sequence numbers of SEQ ID NOs:63, 42 and 43, respectively; the parts without underline are FR1, FR2, FR3 and FR4, respectively, with the sequence numbers of SEQ ID NOs:64, 45, 46 and 47, respectively.

The DNA sequence corresponding to the amino acid sequence of the heavy variable region of anti-OX40 21#H28H33 mAb is:

CAGGTACAGCTGCAGCAGTCAGGTCCAGGACTGGTGAAGCCCTCGCAGACCCTCTCACT
CACCTGTGCCATCTCCGGGGACAATGTCTCTAGCAACGGTGTCTCTTGGGACTGGATCA
GGCAGTCCCCCTCGAGGGGCCTTGAGTGGCTGGGAAGGACATACTATAGGTCCAAGTG
GTATAATGAGTATGCAGTATCTGTGGAAAGTCGAATAACCATCAACCCAGACACATCCA
AGAACCAGTTCTCCCTGCAACTGAACTCTGTGACTCCCGAGGACACGGCTATATATTTC
TGTGTAAGAAATAACTACTTCTTCGATCTCTGGGGCCGTGGTACCCTGGTCACCGTCTCC
TCA (SEQ ID NO: 65)

The amino acid sequence of the light variable region of anti-OX40 21#H28H33 mAb is: 
EIVLTQSPATLSLSPGERATLSCRASQSVSSYLAWYQQKPGQAPRLLIYDASDRATGIPARFS
GSGSGTDFTLTISSLEPEDFAVYYCQLRSNWPPGYTFGQGTKVEIK (SEQ ID NO: 49)
 where the underlined parts are CDR1, CDR2 and CDR3, respectively, with the sequence numbers are SEQ ID NOs: 50-52, respectively; the parts without underline are FR1, FR2, FR3 and FR4, respectively, with the sequence numbers are SEQ ID NOs: 53-56, respectively.

The DNA sequence corresponding to the amino acid sequence of the light variable region of anti-OX40 21#H28H33 mAb is:

GAAATTGTGTTGACACAGTCTCCAGCCACCCTGTCTTTGTCTCCAGGGGAAAGAGCCAC
CCTCTCCTGCAGGGCCAGTCAGAGTGTTAGCAGCTACTTAGCCTGGTACCAACAGAAAC
CTGGCCAGGCTCCCAGGCTCCTCATCTATGATGCATCCGACAGGGCCACTGGCATCCCA

GCCAGGTTCAGTGGCAGTGGGTCTGGGACAGACTTCACTCTCACCATCAGCAGCCTTGA
GCCTGAAGATTTTGCAGTTTATTACTGTCAGCTGCGTAGCAACTGGCCTCCGGGGTACA
CTTTTGGCCAGGGGACCAAGGTGGAGATCAAA (SEQ ID NO: 57)

### 6.2 anti-OX40 21#H65 mAb

The amino acid sequence of the heavy chain variable region of anti-OX40 21#H65 mAb is: 
QVQLQQSGPGLVKPSQTLSLTCAISGDSVSSNSVSWDWIRQSPSRGLEWLGRTYYRSKWYN
EYAVSVENRITINPDTSKNQFSLQLNSVTPEDTAIYFCVRNNYFFDLWGRGTLVTVSS (SEQ
ID NO: 66)
 where the underlined parts are CDR1, CDR2 and CDR3, respectively, (the parts in the box are the mutated parts), with the sequence numbers of SEQ ID NOs:41, 67 and 43, respectively; the parts without underline are FR1, FR2, FR3 and FR4, respectively, with the sequence numbers of SEQ ID NOs:44-47, respectively.

The DNA sequence corresponding to the amino acid sequence of the heavy chain variable region of anti-OX40 21#H65 mAb is:

CAGGTACAGCTGCAGCAGTCAGGTCCAGGACTGGTGAAGCCCTCGCAGACCCTCTCACT
CACCTGTGCCATCTCCGGGGACAGTGTCTCTAGCAACAGTGTCTCTTGGGACTGGATCA
GGCAGTCCCCCTCGAGGGGCCTTGAGTGGCTGGGAAGGACATACTATAGGTCCAAGTG
GTATAATGAGTATGCAGTATCTGTGGAAAATCGAATAACCATCAACCCAGACACATCC

AAGAACCAGTTCTCCCTGCAACTGAACTCTGTGACTCCCGAGGACACGGCTATATATTT
CTGTGTAAGAAATAACTACTTCTTCGATCTCTGGGGCCGTGGTACCCTGGTCACCGTCTC
CTCA (SEQ ID NO: 68)

The amino acid sequence of the light chain variable region of anti-OX40 21#H65 mAb is: 
EIVLTQSPATLSLSPGERATLSCRASQSVSSYLAWYQQKPGQAPRLLIYDASDRATGIPARFS
GSGSGTDFTLTISSLEPEDFAVYYCQLRSNWPPGYTFGQGTKVEIK (SEQ ID NO: 49)
 where the underlined parts are CDR1, CDR2 and CDR3, respectively, with the sequence numbers of SEQ ID NOs: 50-52, respectively; the parts without underline are FR1, FR2, FR3 and FR4, respectively, with the sequence numbers of SEQ ID NOs:53-56, respectively.

The DNA sequence corresponding to the amino acid sequence of the light chain variable region of anti-OX40 21#H65 mAb is:

GAAATTGTGTTGACACAGTCTCCAGCCACCCTGTCTTTGTCTCCAGGGGAAAGAGCCAC
CCTCTCCTGCAGGGCCAGTCAGAGTGTTAGCAGCTACTTAGCCTGGTACCAACAGAAAC
CTGGCCAGGCTCCCAGGCTCCTCATCTATGATGCATCCGACAGGGCCACTGGCATCCCA
GCCAGGTTCAGTGGCAGTGGGTCTGGGACAGACTTCACTCTCACCATCAGCAGCCTTGA
GCCTGAAGATTTTGCAGTTTATTACTGTCAGCTGCGTAGCAACTGGCCTCCGGGGTACA
CTTTTGGCCAGGGGACCAAGGTGGAGATCAAA (SEQ ID NO: 57)

### 6.3 anti-OX40 21#H96 mAb

The amino acid sequence of the heavy chain variable region of anti-OX40 21#H96 mAb is: 
QVQLQQSGPGLVKPSQTLSLTCAISGDSVSSNSVSWDWIRQSPSRGLEWLGRTYYRSKWYN
EYAVSVESRITINPDTSKNQFSLQLNSVTPEDTAIYFCVRNDYFFDLWGRGTLVTVSS (SEQ
ID NO: 69)
 where the underlined parts are CDR1, CDR2 and CDR3, respectively, (the parts in the box are the mutated parts), with the sequence numbers of SEQ ID NOs:41, 42 and 70, respectively; the parts without underline are FR1, FR2, FR3 and FR4, respectively, with the sequence numbers of SEQ ID NOs:44-47, respectively.

The DNA sequence corresponding to the amino acid sequence of the heavy chain variable region of anti-OX40 21#H96 mAb is:

CAGGTACAGCTGCAGCAGTCAGGTCCAGGACTGGTGAAGCCCTCGCAGACCCTCTCACT
CACCTGTGCCATCTCCGGGGACAGTGTCTCTAGCAACAGTGTCTCTTGGGACTGGATCA
GGCAGTCCCCCTCGAGGGGCCTTGAGTGGCTGGGAAGGACATACTATAGGTCCAAGTG
GTATAATGAGTATGCAGTATCTGTGGAAAGTCGAATAACCATCAACCCAGACACATCCA
AGAACCAGTTCTCCCTGCAACTGAACTCTGTGACTCCCGAGGACACGGCTATATATTTC
TGTGTAAGAAATGACTACTTCTTCGATCTCTGGGGCCGTGGTACCCTGGTCACCGTCTC
CTCA (SEQ ID NO: 71)

The amino acid sequence of the light chain variable region of anti-OX40 21#H96 mAb is: 
EIVLTQSPATLSLSPGERATLSCRASQSVSSYLAWYQQKPGQAPRLLIYDASDRATGIPARFS
GSGSGTDFTLTISSLEPEDFAVYYCQLRSNWPPGYTFGQGTKVEIK (SEQ ID NO: 49)
 where the underlined parts are CDR1, CDR2 and CDR3, respectively, with the sequence numbers of SEQ ID NOs: 50-52, respectively; the parts without underline are FR1, FR2, FR3 and FR4, respectively, with the sequence numbers of SEQ ID NOs:53-56, respectively.

The DNA sequence corresponding to the amino acid sequence of the light chain variable region of anti-OX40 21#H96 mAb is:

GAAATTGTGTTGACACAGTCTCCAGCCACCCTGTCTTTGTCTCCAGGGGAAAGAGCCAC
CCTCTCCTGCAGGGCCAGTCAGAGTGTTAGCAGCTACTTAGCCTGGTACCAACAGAAAC
CTGGCCAGGCTCCCAGGCTCCTCATCTATGATGCATCCGACAGGGCCACTGGCATCCCA
GCCAGGTTCAGTGGCAGTGGGTCTGGGACAGACTTCACTCTCACCATCAGCAGCCTTGA
GCCTGAAGATTTTGCAGTTTATTACTGTCAGCTGCGTAGCAACTGGCCTCCGGGGTACA
CTTTTGGCCAGGGGACCAAGGTGGAGATCAAA (SEQ ID NO: 57)

### 6.4 anti-OX40 21#VHnew-L80

The amino acid sequence of the heavy chain variable region of anti-OX40 21#VHnew-L80 is:

QVQLQQSGPGLVKPSQTLSLTCAISGDSVSSNGVSWDWIRQSPSRGLEWLGRTYYRSKWY

NEYAVSVENRITINPDTSKNQFSLQLNSVTPEDTAIYFCVRNDYFFDLWGRGTLVTVSS

(SEQ ID NO: 72)

The underlined parts are CDR1, CDR2 and CDR3, respectively, (the parts in the box are the mutated parts), with the sequence numbers of SEQ ID NOs:63, 67 and 70, respectively; the parts without underline are FR1, FR2, FR3 and FR4, respectively, with the sequence numbers of SEQ ID NOs:44-47, respectively;

The DNA sequence corresponding to the amino acid sequence of the heavy chain variable region of anti-OX40 21#VHnew-L80 is:

CAGGTACAGCTGCAGCAGTCAGGTCCAGGACTGGTGAAGCCCTCGCAGACCCTCTCACT
CACCTGTGCCATCTCCGGGGACAGTGTCTCTAGCAACGGTGTCTCTTGGGACTGGATCA
GGCAGTCCCCCTCGAGGGGCCTTGAGTGGCTGGGAAGGACATACTATAGGTCCAAGTG
GTATAATGAGTATGCAGTATCTGTGGAAAATCGAATAACCATCAACCCAGACACATCC
AAGAACCAGTTCTCCCTGCAACTGAACTCTGTGACTCCCGAGGACACGGCTATATATTT
CTGTGTAAGAAATGACTACTTCTTCGATCTCTGGGGCCGTGGTACCCTGGTCACCGTCT
CCTCA (SEQ ID NO: 73)

The amino acid sequence of the light chain variable region of anti-OX40 21#VHnew-L80 is: 
EIVLTQSPATLSLSPGERATLSCRASQSVGSYLAWYQQKPGQAPRLLIYDASNRATGIPARF
SGSGSGTDFTLTISSLEPEDFAVYYCQQRSNWPPGYTFGQGTKVEIK (SEQ ID NO: 74)
 where the underlined parts are CDR1, CDR2 and CDR3, respectively, (the parts in the box are the mutated parts), with the sequence numbers of SEQ ID NOs:75-77, respectively; the parts without underline are FR1, FR2, FR3 and FR4, respectively, with the sequence numbers thereof are SEQ ID NOs:53-56, respectively;

The DNA sequence corresponding to the amino acid sequence of the light chain variable region of anti-OX40 21#VHnew-L80 is:

GAAATTGTGTTGACACAGTCTCCAGCCACCCTGTCTTTGTCTCCAGGGGAAAGAGCCAC
CCTCTCCTGCAGGGCCAGTCAGAGTGTTGGCAGCTACTTAGCCTGGTACCAACAGAAAC
CTGGCCAGGCTCCCAGGCTCCTCATCTATGATGCATCCAACAGGGCCACTGGCATCCCA
GCCAGGTTCAGTGGCAGTGGGTCTGGGACAGACTTCACTCTCACCATCAGCAGCCTTGA
GCCTGAAGATTTTGCAGTTTATTACTGTCAGCAGCGTAGCAACTGGCCTCCGGGGTACA
CTTTTGGCCAGGGGACCAAGGTGGAGATCAAA (SEQ ID NO: 78)

### 6.5 anti-OX40 21#H96-L80mAb

The amino acid sequence of the heavy chain variable region of anti-OX40 21#H96-L80mAb is: 
QVQLQQSGPGLVKPSQTLSLTCAISGDSVSSNSVSWDWIRQSPSRGLEWLGRTYYRSKWYN
EYAVSVESRITINPDTSKNQFSLQLNSVTPEDTAIYFCVRNDYFFDLWGRGTLVTVSS (SEQ
ID NO: 79)
 where the underlined parts are CDR1, CDR2 and CDR3, respectively, (the parts in the box are the mutated parts), with the sequence numbers of SEQ ID NOs:41, 42 and 70, respectively; the parts without underline are FR1, FR2, FR3 and FR4, respectively, with the sequence numbers of SEQ ID NOs:44-47, respectively;

The DNA sequence corresponding to the amino acid sequence of the heavy chain variable region of anti-OX40 21#H96-L80mAb is:

CAGGTACAGCTGCAGCAGTCAGGTCCAGGACTGGTGAAGCCCTCGCAGACCCTCTCACT
CACCTGTGCCATCTCCGGGGACAGTGTCTCTAGCAACAGTGTCTCTTGGGACTGGATCA
GGCAGTCCCCCTCGAGGGGCCTTGAGTGGCTGGGAAGGACATACTATAGGTCCAAGTG
GTATAATGAGTATGCAGTATCTGTGGAAAGTCGAATAACCATCAACCCAGACACATCCA
AGAACCAGTTCTCCCTGCAACTGAACTCTGTGACTCCCGAGGACACGGCTATATATTTC
TGTGTAAGAAATGACTACTTCTTCGATCTCTGGGGCCGTGGTACCCTGGTCACCGTCTC
CTCA (SEQ ID NO: 80)

The amino acid sequence of the light chain variable region of anti-OX40 21#H96-L80mAb is: 
EIVLTQSPATLSLSPGERATLSCRASQSVGSYLAWYQQKPGQAPRLLIYDASNRATGIPARF

SGSGSGTDFTLTISSLEPEDFAVYYCQQRSNWPPGYTFGQGTKVEIK (SEQ ID NO: 74)
 where the underlined parts are CDR1, CDR2 and CDR3, respectively, (the parts in the box are the mutated parts), with the sequence numbers of SEQ ID NOs:75-77, respectively; the parts without underline are FR1, FR2, FR3 and FR4, respectively, with the sequence numbers of SEQ ID NOs:53-56, respectively;

The DNA sequence corresponding to the amino acid sequence of the light chain variable region of anti-OX40 21#H96-L80mAb is:

GAAATTGTGTTGACACAGTCTCCAGCCACCCTGTCTTTGTCTCCAGGGGAAAGAGCCAC

CCTCTCCTGCAGGGCCAGTCAGAGTGTTGGCAGCTACTTAGCCTGGTACCAACAGAAAC

CTGGCCAGGCTCCCAGGCTCCTCATCTATGATGCATCCAACAGGGCCACTGGCATCCCA

GCCAGGTTCAGTGGCAGTGGGTCTGGGACAGACTTCACTCTCACCATCAGCAGCCTTGA

GCCTGAAGATTTTGCAGTTTATTACTGTCAGCAGCGTAGCAACTGGCCTCCGGGGTACA

CTTTTGGCCAGGGGACCAAGGTGGAGATCAAA (SEQ ID NO:78)

### Embodiment 7: Identification of anti-hOX40 Abs

### 7.1 Binding affinity test of purified anti-hOX40 antibody to hOX40 (ELISA)

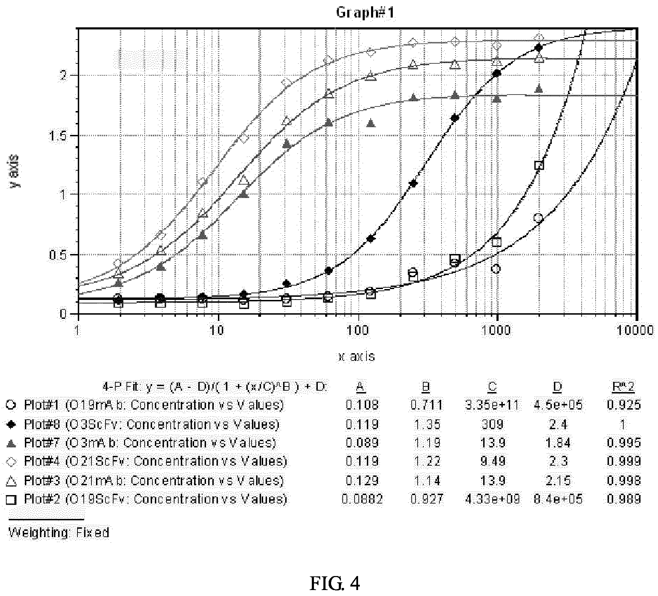

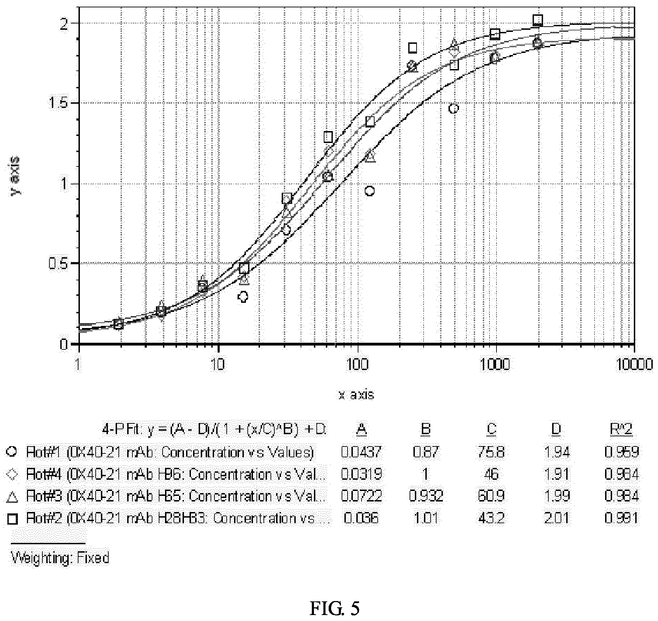

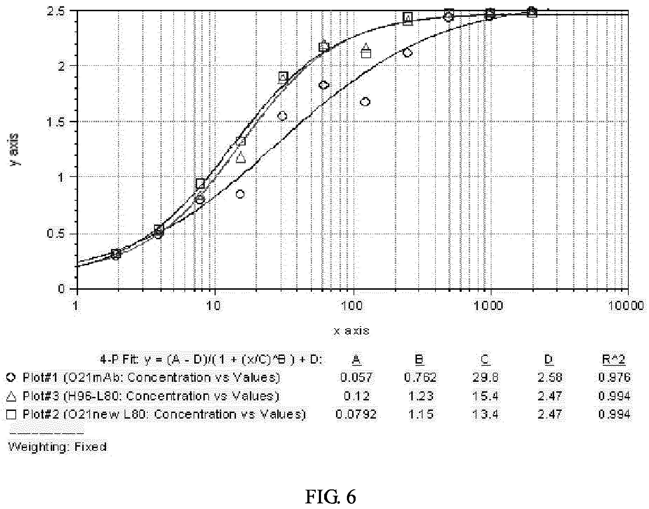

hOX40-muFc was diluted to 2 µg/ml with coating buffer (50 mM Na₂CO₃, NaHCO₃ pH 9.6), 100 µL/well, 4 °C overnight. After washing, blocking was performed using 3% BSA-PBS for 1 h at 37 °C. The anti-hOX40 antibodies were diluted respectively from 2000 ng/ml and was diluted 2-fold in a total of 11 concentrations, with the diluent (1% BSA-PBS) as a control, and incubated at 37 °C for 2h. Goat anti-human IgG-HRP conjugated was added and incubated for 1 h at 37 °C. Soluble one-component TMB substrate developer solution is added, and the developing was performed in dark at room temperature for 5-10min. 2N H₂SO₄ 50 µL/well was added to terminate the color development reaction. The OD of the obtained solution was determined at 450 nm-650 nm on MD SpectraMax Plus 384 microplate reader and SoftMaxPro v5.4 was used for processing data, making graphs and analysis. The results are shown in Figures 4-6. It can be seen from Fig. 4 that the affinity of 019 mAb remains relatively low after the conversion from ScFv to IgG-type antibody; the affinity of O21 mAb does not change much and is good; and the affinity of O3 mAb is significantly improved. It can be seen from Figure 5 and Figure 6 that all the above variants of anti-OX40 21 # mAb can bind to OX40 and the affinity of the *in vitro* affinity-matured anti-OX40 21 # VHnew-L80 mAb and anti-OX40 21#H96-L80 mAb increased approximately two times compared to anti-OX40 21# mAb.

### 7.2 Analysis of Kinetic Affinity Constant of a-hOX40 mAbs to hOX40 by Surface Plasmon Resonance (SPR)

The binding kinetics of anti-hOX40 antibody to recombinant human OX40 was measured by surface plasmon resonance (SRP) method using a BIAcore X100 instrument. Anti-human Fc antibody (no cross-identification against the mouse Fc) was conjugated on CM5 chip, the test antibody was diluted to 5 nM with running buffer and captured as a ligand by the anti-human Fc antibody on the CM5 chip. OX40-muFc was diluted with running buffer to 1000-1.37 nM, in a 2-fold serial dilution factor. The injection time is 180s, the dissociation time is 1800s and the regeneration time is 60s. The running buffer is HBS-EP +, and the regeneration buffer is 10 mM glycine-HCl (pH 2.0). The association rate (kₒₙ) and dissociation rate (k_{off}) were calculated using a simple one-to-one Languir binding model (BIAcore Evaluation Software version 3.2). The equilibrium dissociation constant (kD) is calculated as the ratio k_{off}/kₒₙ. It can be seen from Table 3 that the affinity of the affinity-matured anti-OX40 21 # H96-L80 mAb is increased approximately two times.

**Table 3 Binding kinetic test of anti-hOX40 antibody to hOX40**

| **Name** | **Kon (1/Ms)** | **Koff (1/s)** | **KD (M)** |
|---|---|---|---|
| **03 mAb** | **2.84E+05** | **3.91E-04** | **1.378E-09** |
| **021 mAb** | **4.181E+4** | **6.077E-04** | **1.454E-08** |
| **VHnew-L80** | **3.329E+04** | **3.415E-04** | **1.026E-08** |
| **H96-L80** | **2.999E+04** | **2.368E-04** | **7.898E-09** |

| | | | |
|---|---|---|---|
| *in vitro* agonist activity detection of anti-hOX40 antibody by NF-κb system | | | |

Construction of OX40-CD40 plasmid: the amino acid sequences of the human OX40 extracellular domain and transmembrane domain (i.e., the residue 1 to the residue 235 in P43489) was obtained based on the amino acid sequence of human OX40 (P43489) in the Uniprot protein database; The amino acid sequence of the intracellular domain of human CD40 (i.e., the residue 216 to the residue 277 in P25942) was obtained based on the amino acid sequence of human CD40 (P25942) in the Uniprot protein database; The two amino acid sequences obtained above were spliced together via gene synthesis, then sub-cloned into the commercial vector pcDNA4/myc-HisA (Invitrogen, V863-20) by double digestion by HindIII and EcoRI (Fermentas), and the accuracy of the constructed plasmid was verified by sequencing to obtain recombinant plasmid DNA: pcDNA4-OX40-CD40 (i.e. OX40-CD40 mentioned below);

293T-NF-κB stable cell line: 293T cells were seeded in a 24-well plate (1x10⁵/well and 200 µl DMEM complete medium/well) with 20 µl NF-κB-luciferase-lentivirus (Qiagen, cat: CLS-013L) added, MOI=2. 24 h after infection, the supernatant was discarded and 1 ml of DMEM complete medium was added for continue culturing. After 24 h, the medium was replaced with DMEM complete medium containing 0.3 µg/ml of promycin for continue culturing, the cells were cultured, propagated and frozen for storage. The 293T-NF-κB cells screened by promycin were seeded in a 24-well plate (1x10⁵/well) and stimulated with 10 ng/ml TNF-α for 6 h. Then the cells were lysed and detected for luciferase. It showed that the luciferase values of TNF-α-stimulated cells were significantly higher than those of untreated cells, demonstrating that NF-κB-luciferase has been stably introduced into 293T cells via transgene.

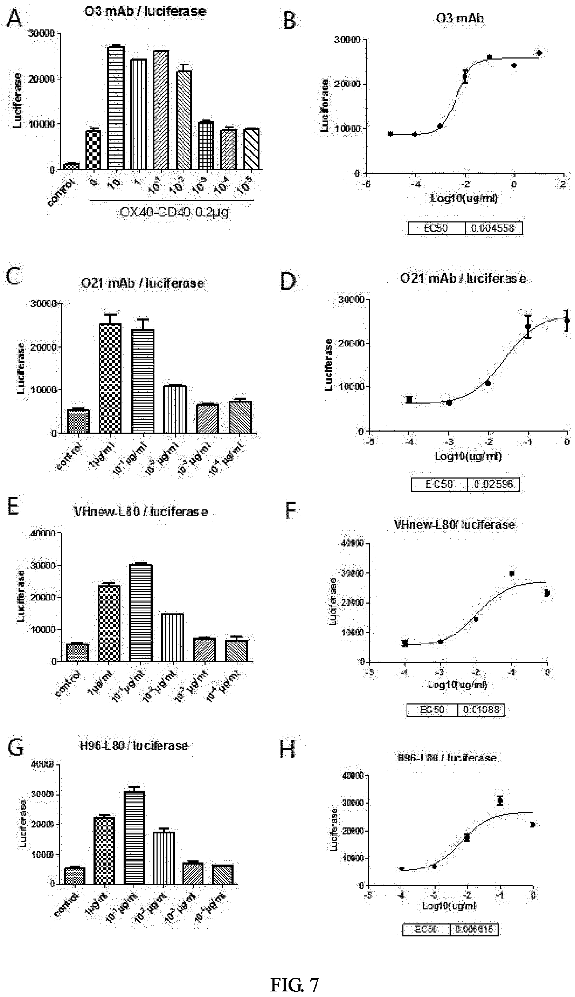

5 x 10⁵ 293T-NF-κB cells were resuspended in medium without double antibiotics and seeded in a 6-well plate. After 24 h, the supernatant was discarded and washed once with PBS. 1.8 ml of culture solution without double antibiotics and serum was added. 0.2 µg of OX40-CD40 plasmid was transfected in the ratio of plasmid: liposome = 1: 3. 4 h after transfection, the supernatant was discarded, replaced with fresh complete culture solution, and the culture was continued. One the day after transfection, the cells were seeded in a 96-well plate at a density of 5 × 10⁴/well, and antibodies with a series of concentration gradients (initial concentration of 10 µg/ml, seven gradients through 10-fold dilutions) were added, at the same time, cross-link (Jackson ImmunoResearch Laboratories: 109-006-008) with the same concentration was added and the culture was continued for 6 h, then the cells were lysed and detected for luciferase. As shown in Figure 7, A, C, E and G represent the results of the *in vitro* agonist activity detection of O3 mAb, O21 mAb, anti-OX40 21 # VHnew-L80 mAb and anti-OX40 21 # H96-L80 mAb at different concentrations, respectively; B, D, F and H are the results of the *in vitro* agonist activity detection after logistic transformation, respectively. The results show that both O3 mAb and O21 mAb have good agonist activity *in vitro* and exhibit good dose-dependency. The agonist activity of anti-OX40 21 # VHnew-L80 mAb and anti-OX40 21 # H96-L80 mAb with improved affinity is increased approximately two times.

### Embodiment 8 Effects of different subtypes on antibody agonist activity

Various variants of anti-OX40 21 # mAb have a high affinity for hOX40 and good binding kinetics to hOX40. The agonist activity of one of them, anti-OX40 21 # H96-L80 mAb is increased approximately two times. Therefore, the present application is further illustrated by taking the anti-OX40 21 # H96-L80 mAb (hereinafter referred to as H96-L80) as an example, and the rest of the variants will not be described in detail. However, it should be noted that in the following embodiments, although only H96-L80 is described, it should not be understood that the other variants of anti-OX40 21 # mAb do not have the corresponding characteristics, or the other two antibodies (O3 mAb and 019 mAb) do not have the corresponding characteristics. Further experimental data for H96-L80 in each of the following embodiments are only intended to demonstrate that the various anti-OX40 antibodies obtained according to the methods herein disclosed have high activity, affinity, functionality, or stability.

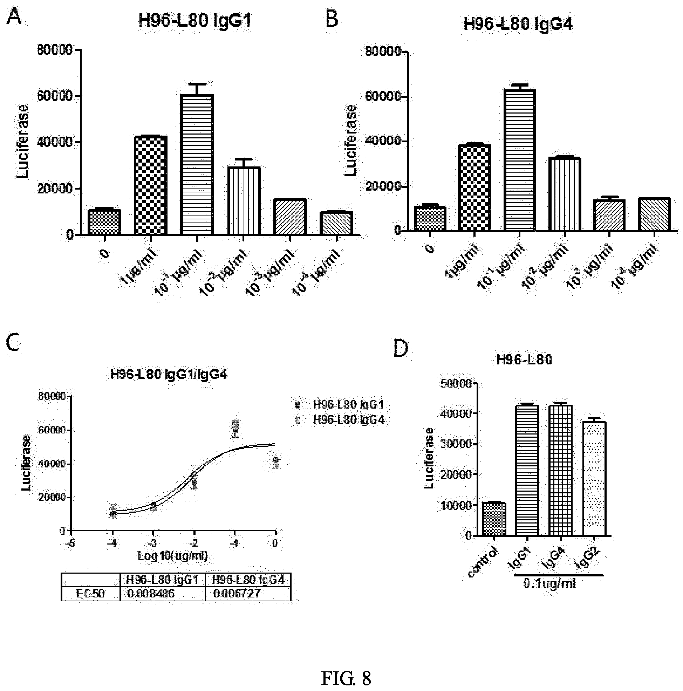

The amino acid sequences of human IgG1 and IgG2 constant regions were obtained based on the amino acid sequences of the constant regions of human immunoglobulin gamma1 (IgG1) and human immunoglobulin gamma2 (IgG2) (P01857 and P01859 respectively) in the Uniprot protein database. The rest steps were performed as described above to give anti-OX40 21#H96-L80 IgG1 mAb and anti-OX40 21#H96-L80 IgG2 mAb. The experiment was carried out according to the method in Embodiment 7, and the results are shown in Figure 8, in which Figure 8A and Figure 8B show the results of *in vitro* agonist activity detection at different concentrations for IgG1 and IgG4 subtypes respectively; Figure 8C shows the comparison of IgG1 and IgG4 subtypes after logistic transformation; Figure 8D shows that the antibody agonist activities of the three subtypes of H96-L80 are comparable at 0.1 µg/ml.

### Embodiment 9: Detection of Binding Ability of Purified H96-L80 Antibodies to CD4 + T and CD8 + T Cells after Activation of Human and Rhesus

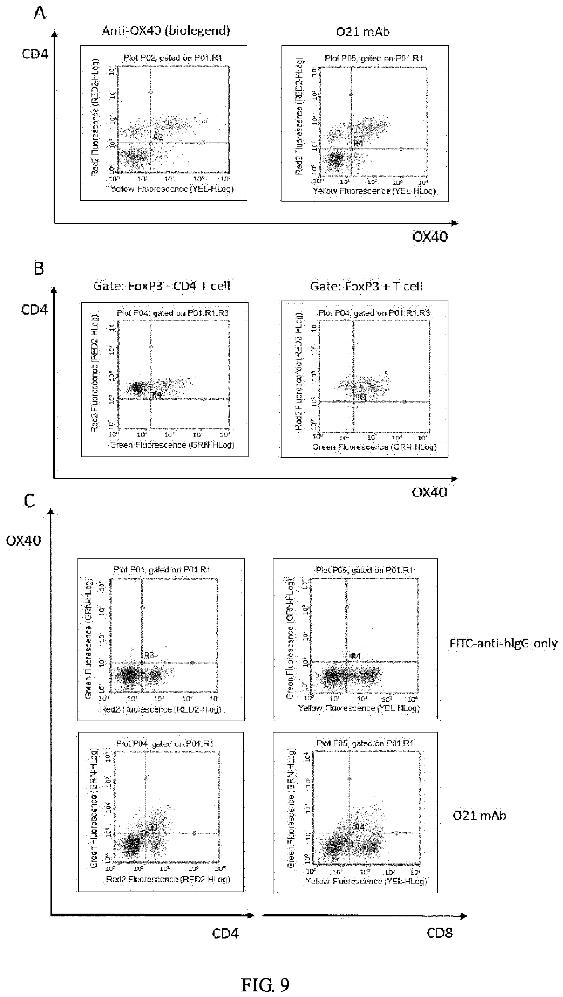

Peripheral blood mononuclear cells (PBMCs) were isolated from peripheral blood leukocytes of healthy donors by density gradient centrifugation using human lymphocyte separation fluid (Tianjin Hao Yang) and seeded into RPMI complete medium. PBMCs were activated with PHA at a final concentration of 5 µg/ml for 48 hours. The cells were resuspended in 0.5% PBS-BSA Buffer, H96-L80 proteins were added and the mixture was incubated on ice for 20 min. After washing, secondary antibody FITC anti-hIgG (Cat # 409310) or antibody PE anti-hOX40 (Cat # 350003) and antibody APC anti-human CD4 (Cat # 317416) from Biolegend were added and incubated on ice for 20 min. After washing, the cells were resuspended in 500 µl of 0.5% PBS-BSA Buffer and detected by flow cytometry. Results are shown in Figure 9A, H96-L80 binds well to activated CD4 + T cells.

To test the binding ability of H96-L80 to Tregs and Teff in CD4 + cells, the activated human PBMCs above were stained. The cells were resuspended in 0.5% PBS-BSA Buffer, H96-L80 proteins were added and the mixture was incubated on ice for 20 min. After washing, secondary antibody FITC anti-hIgG and antibody APC anti-human CD4 from Biolegend were added and the obtained mixture was incubated on ice for 20 min. After washing, the transmembrane fixative (BD, 51-2090 KZ) was applied for 1 h and the obtained mixture was washed using transmembrane solution (eBioscience, 00-8333-56) and then resuspended in transmembrane solution. PE anti-human Foxp3 (Cat # 320208) was added and stained overnight at 4 °C. After washing, the cells were resuspended in 500 µl of 0.5% PBS-BSA Buffer and detected by flow cytometry. Results are shown in Figure 9B, H96-L80 binds well to activated CD4+Foxp3+Treg and CD4+Foxp3-Teff cells.

To test the binding ability of H96-L80 to rhesus OX40, activated rhesus peripheral blood mononuclear cells were obtained using the above procedure. The cells were resuspended in 0.5% PBS-BSA Buffer, H96-L80 proteins were added and the mixture was incubated on ice for 20 min. After washing, secondary antibody FITC anti-hIgG or antibody APC anti-human CD4 and PE anti-human CD8a (Cat#301008) from Biolegend were added and incubated on ice for 20 min. After washing, the cells were resuspended in 500 µl of 0.5% PBS-BSA Buffer and detected by flow cytometry. Results are shown in Figure 9C, H96-L80 well binds to activated rhesus CD4 + T and CD8 + T cells, suggesting that H96-L80 can bind to rhesus OX40.

### Embodiment 10: Anti-OX40 Antibody Promotes Activation and Proliferation of T Cells

10.1 The activation and proliferation of T-cells were used to study the *in vitro* activity of anti-OX40 scFv antibodies and evaluate their agonist function.

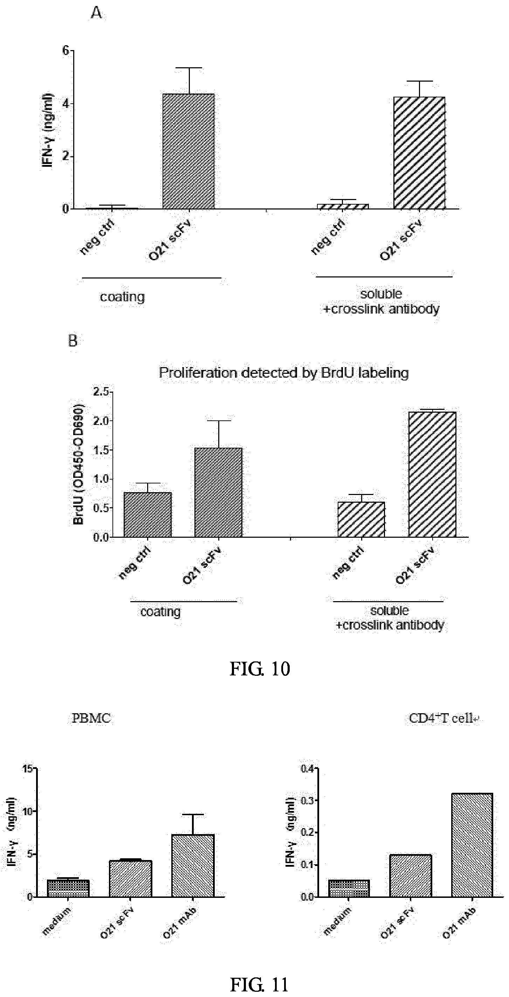

Peripheral blood mononuclear cells (PBMCs) were isolated from peripheral blood leukocytes of healthy donors by density gradient centrifugation using human lymphocyte separation fluid (Tianjin Hao Yang) and seeded into RPMI complete medium. 96-well plates were pre-coated with 50 µl of 1 µg/ml anti-CD3 overnight at 4 °C. The plates in the experimental group was coated with 50 µl of 2 µg/ml O21 scFv at 37 °C for 2 h, at the same time, soluble O21 scFv with a final concentration of 2 µg/ml and cross-link (Jackson ImmunoResearch Laboratories: 109-006-008) with a final concentration of 4 µg/ml were added. The negative control was RPMI complete medium. The amount of PBMCs was 2×10⁵/well, the cells were cultured for five days and then the supernatant was taken. As shown in Figure 10, the level of IFN-γ in the supernatant was detected by the IFN-γ ELISA detection kit (Figure 10A) and the proliferation of T cells was detected by the BrdU staining kit (Roche: 11647229001). It can be seen that O21 scFv has good activity of activating PBMCs and promoting the proliferation of T cells under both coating and cross-link administration modes.

### 10.2 The agonist activities of O21 scFv and IgG were assessed through in vitro activation of PBMCs and CD4 + T cells

Peripheral blood mononuclear cells (PBMCs) were isolated from peripheral blood leukocytes of healthy donors by density gradient centrifugation using human lymphocyte separation fluid (Tianjin Hao Yang) and seeded into RPMI complete medium. CD4 + T cells were isolated from PBMCs using CD4 + T Cell Isolation Kit (Miltenyi, cat # no. 130-096-533). 96-well plates were pre-coated with 50 µl of 1 µg/ml anti-CD3 overnight at 4°C. The plates in the experimental group was coated with 50 µl of 2 µg/ml O21 scFv or O21 mAb at 37 °C for 2 h and the negative control was the same dose of hIgG-Fc. The amount of PBMCs and CD4 + T cells was 2×10⁵/well, and the supernatant was collected after five days of culture. The level of IFN-γ in the supernatant was detected by IFN-γ ELISA kit (ebioscience).

As shown in Figure 11, it can be seen that O21 mAb has better agonist activity than O21 scFv for PBMCs (Figure 11A) and CD4 + T cells (Figure 11B).

### Embodiment 11: Effect of IgG1 and IgG4 Subtypes of Anti-OX40 Antibody H96-L80 on T-cell Subsets

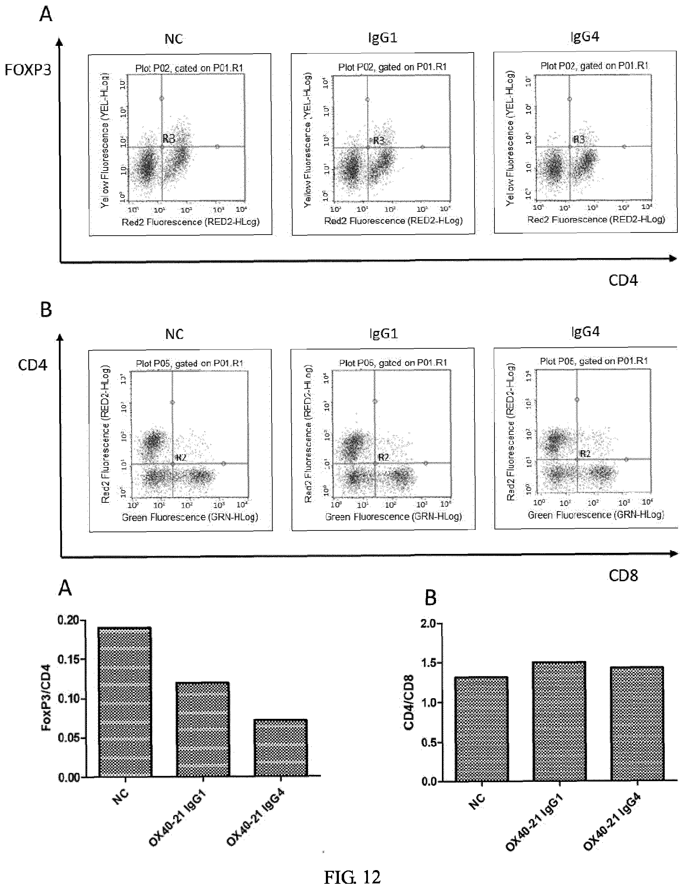

Human peripheral blood mononuclear cells (PBMCs) were obtained according to Embodiment 10 and PBMCs were stimulated with dissolved anti-CD28 (0.5 µg/ml) and plate-bound anti-CD3 (3 µg/ml), and anti-human OX40 mAb H96-L80 IgG1 or IgG4 (10 µg/ml). After 48 hours, the cells were harvested. The cells were stained with antibody APC anti-human CD4 and PE anti-human CD8a (Cat # 301008) from Biolegend or stained with antibody APC anti-human CD4 and PE anti-human Foxp3 (Cat # 320208) from Biolegend, and then detected by flow cytometry. Results are shown in the figure below. Both the O21 mAb H96-L80 IgG1 and IgG4 subtypes reduce the proportion of CD4 + Foxp3 + Tregs in CD4 + T cells (Figure 12A) but do not affect the ratio of CD4 + T to CD8 + T cells (Figure 12B).

### Embodiment 12: Inhibition of Tumor Growth by Anti-OX40 Antibody in Mice

### 12.1 The NOD-SCID mouse tumor model implanted with tumor cells PC-3 and human PBMCs was used to evaluate the in vivo efficacy of anti-OX40 antibody

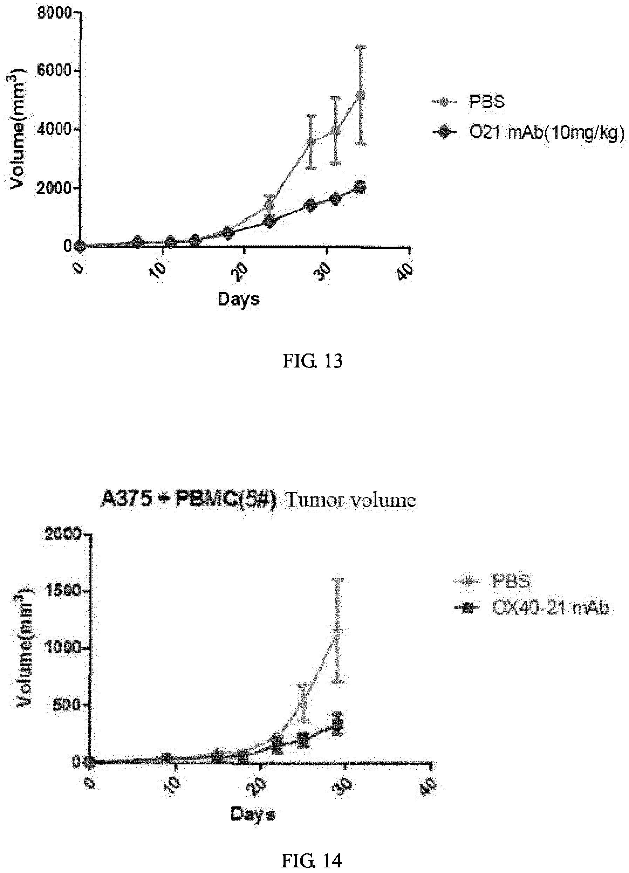

Mice were injected subcutaneously (SC) with PC-3 (ATCC CRL-1435tm) and human peripheral blood mononuclear cells (PBMCs) on day 0 and injected intraperitoneally with 10 mg/kg 021 mAb or PBS on days 0 and 7, PBS was used as a negative control, each group comprised 5 mice. Tumor formation was observed twice a week and the major and minor diameters of the tumors were measured with a vernier caliper. The tumor volume was calculated, and the tumor growth curve was plotted. The results are shown in Figure 13, and it can be seen that the antibody O21 mAb can significantly inhibit tumor growth.

### 12.2 The NOD-SCID mouse tumor model implanted with tumor cells A375 and human PBMCs was used to evaluate the in vivo efficacy of anti-OX40 antibody

Mice were injected subcutaneously (SC) with 7×10⁶ of A375 (ATCC CRL-1619^{™}) and 1×10⁶ of human peripheral blood mononuclear cells (PBMCs) on Day 0 and injected intraperitoneally with 1 mg/kg O21 mAb or PBS on Day 0 and Day 7, PBS was used as a negative control, each group comprised 5 mice. Tumor formation was observed twice a week and the major and minor diameters of the tumors were measured with a vernier caliper. The tumor volume was calculated, and the tumor growth curve was plotted. It can be seen in Figure 14 that the antibody O21 mAb can significantly inhibit tumor growth.

### Embodiment 13: Stability Test of Anti-OX40 Abs

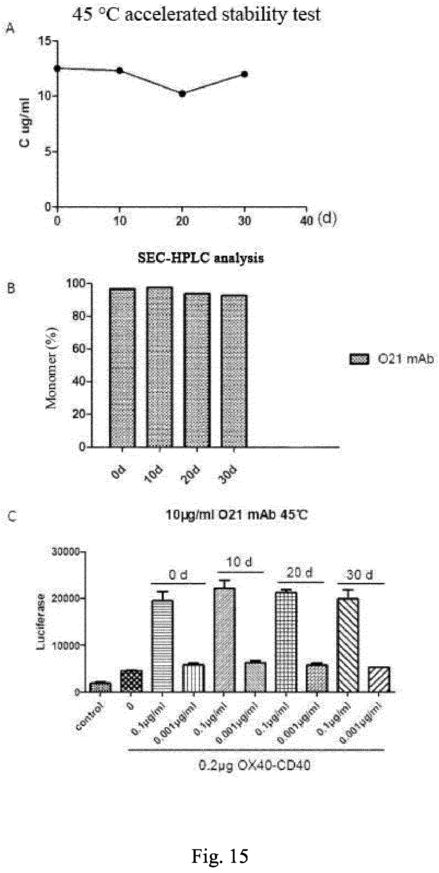

The stability of the anti-hOX40 antibody O21 mAb was tested using an accelerated stability test at 45 °C. The specific experimental method is as follows: the anti-O21 mAb antibody was concentrated to about 10 mg/ml, placed in a 45 °C water bath, and sampled on Day 0, Day 10, Day 20 and Day 30 for concentration, SEC-HPLC and NF-κb analysis experiment. SEC-HPLC analysis experiment was performed using Shimadzu LC20ATHPLC liquid chromatograph; the sample was concentrated to 1mg/ml, the flow rate was 0.5 ml/min, the total sample volume was 50 µg; after sample loading, isocratic elution was performed for 30 min; NF -κb analysis experiment was performed as in Embodiment 7, and the results are shown in Figure 15, it can be seen that anti-hOX40 antibody O21 mAb has good stability *in vitro.*

### Embodiment 14 Pharmacokinetic Evaluation of Anti-hOX40 Antibody O21 mAb

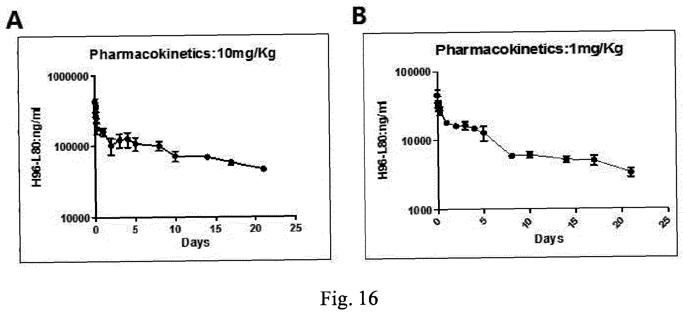

The pharmacokinetics of the H96-L80 IgG1 antibody was evaluated at a dose of 10 mg/kg and 1 mg/kg. Balb/C mice (female, 8-week-old) were used, 16 mice were selected, and each dose was divided into A/B groups, with each group comprising four mice. All the mice were injected intravenously with 200 µg (10 mg/kg) or 20 µg (1 mg/kg) of the H96-L80 IgG1 antibody. After administration, 100 µl of blood was taken at 14 points in time respectively. One group of mice was taken at each time point, two groups took turns. Serum was separated. The concentration of the test protein in serum was determined by ELISA: plates were coated with hOX40-muFc, serum samples with appropriate degree of dilution were added and Goat anti-Human IgG HRP (Sigma Cat NO: A0170) was added and color development test was performed using TMB. H96-L80 IgG1 antibody was used as a standard protein for making a standard curve. Pharmacokinetic parameters were calculated using WinNolin software. The mean C-T curve is shown in Figure 16. The H96-L80 IgG1 antibody of the present invention is relatively stable. The half-life *in vivo* thereof averages 205 hours at a dose of 1 mg/kg and the half-life *in vivo* thereof averages 371 hours at a dose of 10mg/kg. After detection, there are no production of antibodies against anti-H96-L80 antibody.

## Claims

1. A complete anti-OX40 antibody, comprising
a heavy chain variable region having a sequence as set forth in SEQ ID NO: 79, and
a light chain variable region having a sequence as set forth in SEQ ID NO: 74.

2. A composition, comprising the complete anti-OX40 antibody according to claim 1 , and optionally a pharmaceutically acceptable carrier or excipient, and optionally an additional bioactive substance.

3. The complete anti-OX40 antibody according to claim 1, or the composition according to claim 2, for use in preventing or treating tumors.

## Patentansprüche

1. Vollständiger Anti-OX40-Antikörper, umfassend
eine variable Region der schweren Kette, die eine Sequenz aufweist, wie in SEQ ID NO: 79 angegeben, und
eine variable Region der leichten Kette, die eine Sequenz aufweist, wie in SEQ ID NO: 74 angegeben.

2. Zusammensetzung, umfassend den vollständigen Anti-OX40-Antikörper nach Anspruch 1, und optional einen pharmazeutisch verträglichen Träger oder Hilfsstoff, und optional eine zusätzliche bioaktive Substanz.

3. Vollständiger Anti-OX40-Antikörper nach Anspruch 1 oder Zusammensetzung nach Anspruch 2 zur Verwendung bei einem Vorbeugen oder einem Behandeln von Tumoren.

## Revendications

1. Anticorps anti-OX40 complet, comprenant
une région variable de chaîne lourde comprenant une séquence telle que présentée dans SEQ ID NO : 79, et
une région variable de chaîne légère comprenant une séquence telle que présentée dans SEQ ID NO : 74.

2. Composition, comprenant l'anticorps anti-OX40 complet selon la revendication 1, et éventuellement un véhicule ou excipient pharmaceutiquement acceptable, et éventuellement une substance bioactive supplémentaire.

3. Anticorps anti-OX40 complet selon la revendication 1, ou composition selon la revendication 2, pour une utilisation dans la prévention ou le traitement de tumeurs.
